# FASCICULE DE BREVET EUROPEEN

(11) **EP 2 247 582 B1**
(45) Date de publication et mention de la délivrance du brevet: **07.09.2016**
(21) Numéro de dépôt: 09722321.8
(22) Date de dépôt: 15.01.2009
(51) Int. Cl.: C07D 305/14

(54) **FORME CRISTALLINE ANHYDRE DU DIMÉTHOXY DOCÉTAXEL ET SES PROCÉDÉS DE PRÉPARATION**
ANHYDRISCHE KRISTALLINE FORM VON DIMETHOXYDOCETAXEL UND VERFAHREN ZU IHRER HERSTELLUNG
ANHYDROUS CRYSTALLINE FORM OF DIMETHOXY DOCETAXEL AND METHODS FOR PREPARING SAME

(30) Priorité: 17.01.2008 FR 0800243
(43) Date de publication de la demande: 10.11.2010
(60) Demande divisionnaire: 14150580.0
(73) Titulaire: Aventis Pharma S.A., 92160 Antony (FR)
(72) Inventeur: BILLOT, Pascal, F-75013 Paris (FR); DUFRAIGNE, Marielle, F-75013 Paris (FR); ELMALEH, Hagit, F-75013 Paris (FR); GUILIANI, Alexandre, F-75013 Paris (FR); MANGIN, Fabrice, F-75013 Paris (FR); RORTAIS, Patricia, F-75013 Paris (FR); ZASKE, Lionel, F-75013 Paris (FR)
(74) Mandataire: Gaslonde, Aude
(86) Numéro de dépôt international: PCT/FR2009/000042
(87) Numéro de publication internationale: WO 2009/115655

(56) Documents cités:
- WO-A-96/30355
- WO-A-2005/028462
- CAIRA M R: "CRYSTALLINE POLYMORPHISM OF ORGANIC COMPOUNDS" TOPICS IN CURRENT CHEMISTRY, SPRINGER, BERLIN, DE, vol. 198, 1 janvier 1998 (1998-01-01), pages 163-208, XP001156954 ISSN: 0340-1022

## Description

La présente invention concerne une forme cristalline du diméthoxy docétaxel ou (2R,3S)-3-tert-butoxycarbonylamino-2-hydroxy-3-phénylpropionate de 4-acétoxy-2a-benzoyloxy-5β,20-époxy-1-hydroxy-7β,10β-diméthoxy-9-oxo-tax-11-ène-13α-yle, et ses procédés de préparation.

Le (2R,3S)-3-tert-butoxycarbonylamino-2-hydroxy-3-phénylpropionate de 4-acétoxy-2a-benzoyloxy-5β,20-époxy-1-hydroxy-7β,10β-diméthoxy-9-oxo-tax-11-ène-13α-yle présente des propriétés anticancéreuses et antileucémiques remarquables.

Le (2R,3S)-3-tert-butoxycarbonylamino-2-hydroxy-3-phénylpropionate de 4-acétoxy-2a-benzoyloxy-5β,20-époxy-1-hydroxy-7β,10β-diméthoxy-9-oxo-tax-11-ène-13α-yle est préparé selon le procédé qui est décrit plus particulièrement dans la demande internationale PCT WO 96/30355 ou la demande internationale PCT WO 99/25704. Selon le procédé décrit dans ces demandes le produit n'est pas cristallisé et n'est pas caractérisé.

Il a été trouvé que le solvat acétonique du (2R,3S)-3-ter-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle (dénommé forme A) était parfaitement déterminé et caractérisé selon le brevet publié sous le numéro WO2005/028462.

La présente description concerne de nouvelles formes cristallines à l'exclusion de la forme acétonate seule connue à ce jour.

Selon la présente description il a été maintenant trouvé que certaines formes anhydres, certains solvats -ou hétéro-solvats- éthanoliques et des formes hydratées sont parfaitement caractérisées d'un point de vue structure physique et chimique.

Parmi les formes anhydres du (2R,3S)-3-ter-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle cinq formes différentes ont été identifiées (dont une selon l'invention), parmi les solvats -ou hétéro-solvats- éthanoliques du (2R,3S)-3-ter-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle quatre formes différentes ont été identifiées et parmi les hydrates du (2R,3S)-3-ter-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13a-yle deux formes différentes ont été identifiées.

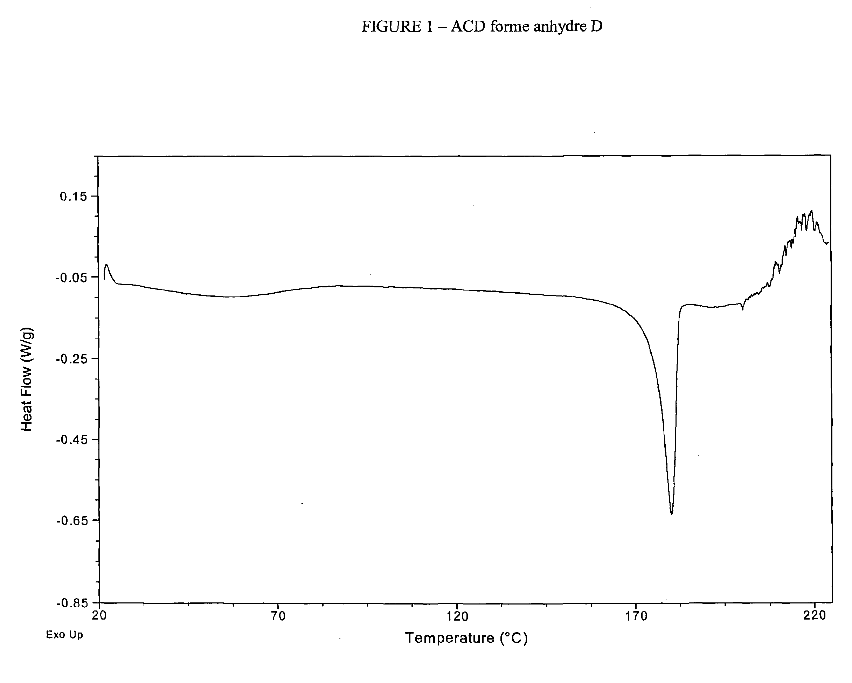

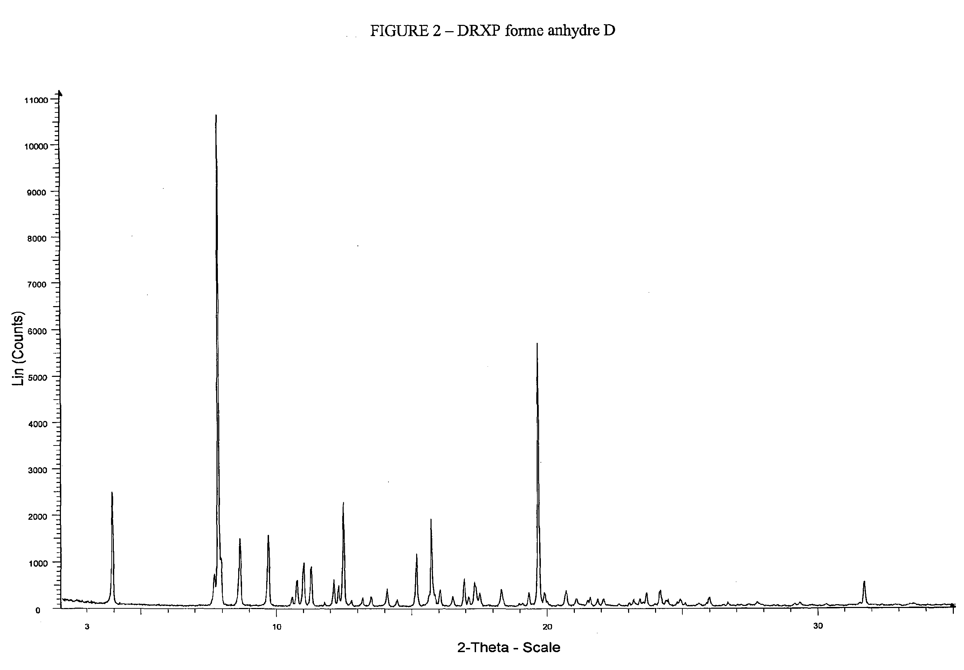

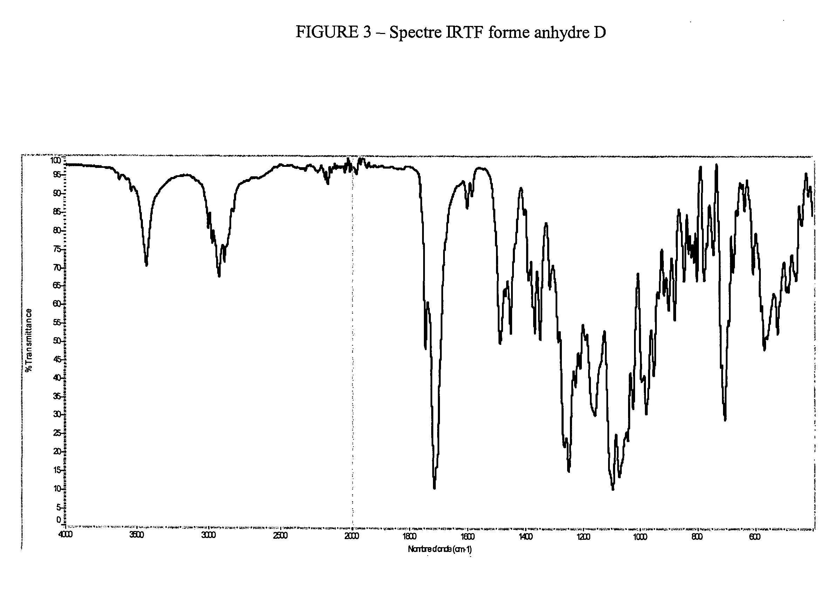

Les cinq formes anhydres identifiées ont été obtenues selon les procédés suivants :
- La forme anhydre B (hors invention) par un procédé qui consiste à chauffer entre 100 et 110°C sous vide ou balayage d'azote la forme acétonique ou forme A obtenue selon le brevet mentionné ci-dessus. Ce traitement est de préférence réalisé pendant au moins 9 heures avant retour à température ambiante sans induire de dégradation chimique. Son point de fusion par ACD est d'environ 150 °C. Le diagramme DRXP de la forme anhydre B présente des raies caractéristiques situées à 7.3, 8.1, 9.8, 10.4, 11.1, 12.7, 13.1, 14.3, 15.4 et 15.9 ± 0.2 degrés 2-Thêta.
- La forme anhydre C (hors invention) est obtenue par maturation de la forme A solvat à l'acétone, ou de la forme anhydre B, dans l'eau suivie d'un séchage jusqu'à environ 50°C et maintien entre 0 et 5% HR à température ambiante. Son point de fusion par ACD est d'environ 146 °C. Le diagramme DRXP de la forme anhydre C présente des raies caractéristiques situées à 4.3, 6.8, 7.4, 8.7, 10.1, 11.1, 11.9, 12.3, 12.6 et 13.1 ± 0.2 degrés 2-Thêta. C'est parmi les différentes formes anhydres la moins stable de toutes les formes décrites dans la présente description. Elle évolue en présence d'une humidité relative supérieure à 5% vers une forme hydratée.
- La forme anhydre D (selon l'invention) est obtenue selon un premier procédé par cristallisation de la forme A dans une huile (notamment le Miglyol) suivie d'un rinçage avec un alcane, par exemple l'heptane. Le deuxième procédé de préparation consiste à laisser cristalliser pendant environ 48 heures une solution du (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle dans un mélange de polysorbate 80 pH 3.5, d'éthanol et d'eau (de préférence un mélange 25/25/50). Son point de fusion par ACD est d'environ 175 °C (*cf.* Figure 1) et s'avère le plus élevé de toutes les formes anhydres isolées. Le diagramme DRXP de la forme anhydre D (*cf.* Figure 2) présente des raies caractéristiques situées à 3.9, 7.7, 7.8, 7.9, 8.6, 9.7, 10.6, 10.8, 11.1 et 12.3 ± 0.2 degrés 2-Thêta. Le spectre IRTF de la forme anhydre D présente des bandes caractéristiques situées à 979, 1072, 1096, 1249, 1488, 1716, 1747, 3436 ± 1 cm⁻¹ (*cf.* Figure 3). C'est la forme anhydre la plus stable parmi toutes les formes décrites dans la présente description.
- La forme anhydre E (hors invention) est obtenue à température ambiante par maturation de la forme acétonique ou forme A dans l'éthanol de façon à former intermédiairement une forme éthanolique qui est ensuite désolvatée sous balayage d'azote ou par chauffage à environ 100°C pendant 2 heures. Son point de fusion par ACD est d'environ 157 °C. Le diagramme DRXP de la forme anhydre E présente des raies caractéristiques situées à 7.1, 8.1, 8.9, 10.2, 10.8, 12.5, 12.7, 13.2, 13.4 et 13.9 ± 0.2 degrés 2-Thêta.
- La forme anhydre F (hors invention) est obtenue par désolvatation de l'hétéro-solvat éthanol/eau à 120°C sous atmosphère d'azote pendant 24 heures puis maintien sous ambiance sèche à 0% HR à température ambiante. Son point de fusion par ACD est d'environ 148 °C. Le diagramme DRXP de la forme anhydre F présente des raies caractéristiques situées à 4.4, 7.2, 8.2, 8.8, 9.6, 10.2, 10.9, 11.2, 12.1 et 12.3 ± 0.2 degrés 2-Thêta.

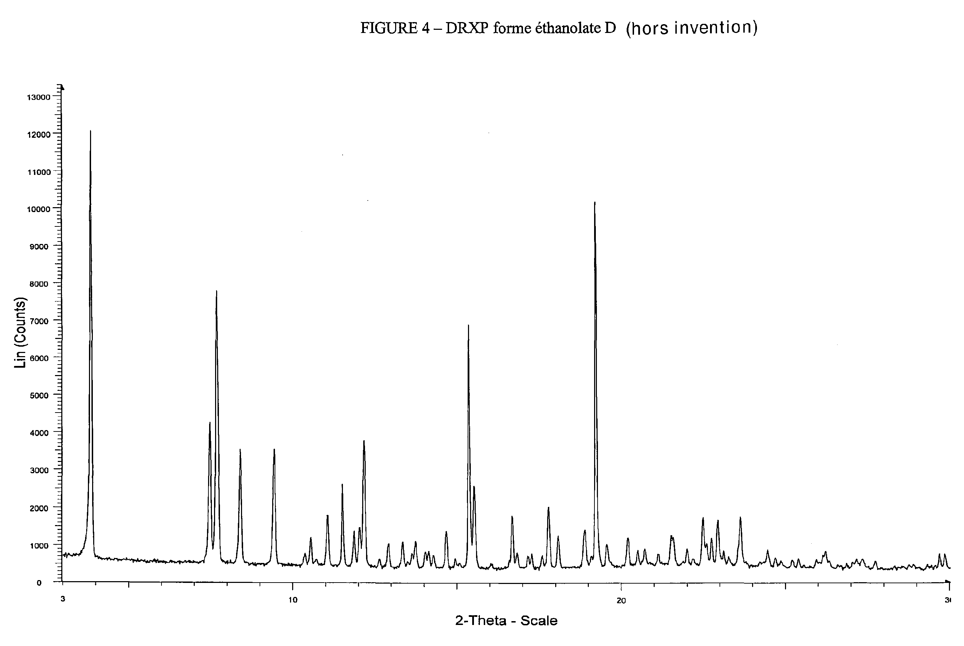

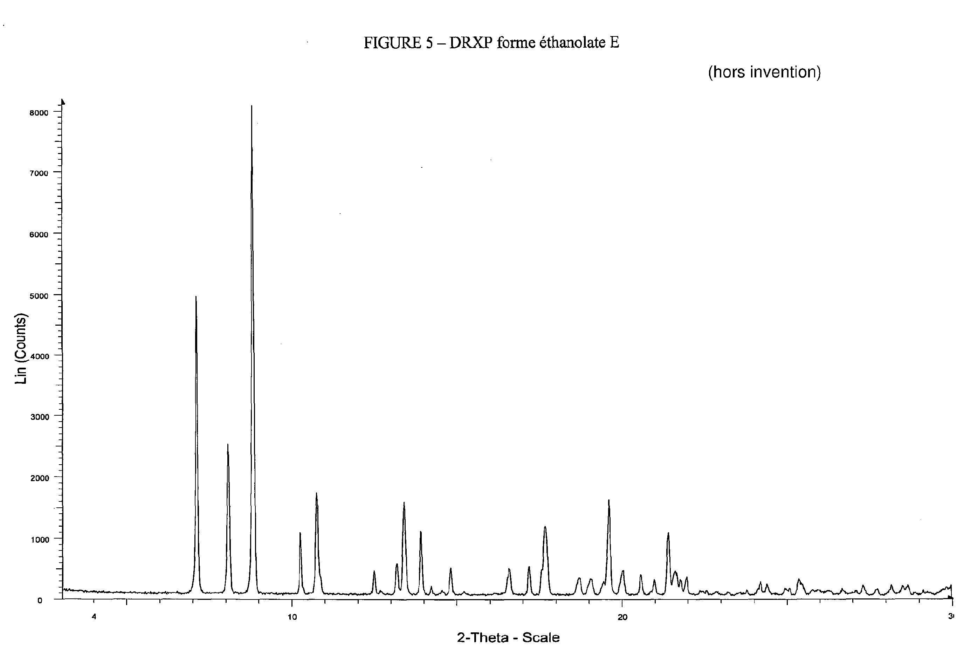

Les formes cristallines identifiées sous forme de solvat ou d'hétéro-solvat à l'éthanol et qui ne font pas partie de l'invention sont au nombre de quatre :
- La forme éthanolate B est obtenue à température ambiante par maintien de la forme anhydre B sous ambiance saturée en vapeurs d'éthanol. Le diagramme DRXP de la forme éthanolate B présente des raies caractéristiques situées à 7.3, 7.8, 8.8, 10.2, 12.6, 12.9, 13.4, 14.2, 14.7 et 15.1 ± 0.2 degrés 2-Thêta.
- La forme éthanolate D est obtenue à température ambiante par maintien de la forme anhydre D sous ambiance saturée en vapeurs d'éthanol. Le diagramme DRXP de la forme éthanolate D (*cf.* Figure 4) présente des raies caractéristiques situées à 3.8, 7.5, 7.7, 8.4, 9.4, 10.3, 10.5, 11.1, 11.5 et 11.9 ± 0.2 degrés 2-Thêta.
- La forme éthanolate E est obtenue à température ambiante par maturation de la forme acétonate A dans l'éthanol. Le diagramme DRXP de la forme éthanolate E (*cf.* Figure 5) présente des raies caractéristiques situées à 7.1, 8.1, 8.8, 10.2, 10.7, 12.5, 13.2, 13.4, 13.9 et 14.2 ± 0.2 degrés 2-Thêta.
- La forme F hétéro-solvat éthanol/eau est obtenue par maintien de la forme B dans une quantité minimale d'éthanol au reflux, refroidissement lent et isolement à température et humidité relative ambiantes. Le diagramme DRXP de la forme F hétéro-solvat éthanol/eau présente des raies caractéristiques situées à 4.4, 7.2, 8.2, 8.3, 8.8, 9.6, 10.3, 10.9, 11.2 et 12.2 ± 0.2 degrés 2-Thêta.

Les formes cristallines identifiées sous forme d'hydrate et qui ne font pas partie de l'invention sont au nombre de
deux :
- Les formes C hydratées sont obtenues à température ambiante par maintien de la forme anhydre C dans une atmosphère contenant au moins 10% d'humidité relative. Le diagramme DRXP de la forme monohydrate C présente des raies caractéristiques situées à 4.3, 6.8, 7.4, 8.6, 10.1, 11.1, 11.9, 12.2, 12.6 et 13.3 ±0.2 degrés 2-Thêta.
- La forme C dihydrate est obtenue à température ambiante par maintien de la forme anhydre C dans une atmosphère contenant au moins 60% d'humidité relative. Le diagramme DRXP de la forme C dihydrate présente des raies caractéristiques situées à 4.2, 6.9, 7.5, 8.4, 9.9, 10.9, 11.7, 12.3, 12.6 et 13.2 ± 0.2 degrés 2-Thêta.

D'autres solvats, non éthanoliques, de la forme B ont été préparés comme notamment ceux obtenus avec les solvants suivants : dichlorométhane, diisopropylether, n-propanol, isopropanol, toluène, méthylisobutyl cétone, tétrahydrofuranne, diméthylformamide, acétate d'ethyle...

La présente invention sera plus complètement décrite à l'aide des exemples suivants qui ne doivent pas être considérés comme limitatifs de l'invention.

### Conditions expérimentales d'analyse :

### Analyse Calorimétrique Différentielle (ACD) :

Les mesures ont été effectuées sur un analyseur thermique DSC2010 T.A. Instruments. L'échantillon est soumis à une programmation de température de 25°C à 225°C avec une vitesse de chauffe de 5°C/min. Le produit est placé dans une capsule en aluminium sertie et la quantité de produit analysé est comprise entre 2 et 5 mg. Un balayage constant d'azote de 55 mL/min est utilisé au niveau de l'enceinte du four.

### Diffraction des Rayons X par la poudre (DRXP) :

Les analyses ont été réalisées sur un diffractomètre Panalytical X'Pert Pro présentant un montage en réflexion à géométrie focalisante de type Bragg-Brentano (*θ*-2*θ*). Le produit analysé est déposé en couche mince sur un monocristal de silicium. Un tube à anticathode de cuivre (45kV/40mA) fournit un rayonnement incident Cu *Kα*₁ (λ = 1,5406 Å). Le faisceau est collimaté à l'aide de fentes de Sollers qui améliorent le parallélisme et de fentes variables qui limitent la diffusion. Un détecteur X'Celerator complète le dispositif. Les caractéristiques d'enregistrement des diagrammes sont les suivantes: balayage de 2 à 30 degrés en 2*θ*, temps de comptage de 100 à 500 secondes par pas avec un pas de 0.017°.

### Spectrométrie Infra-Rouge à Transformée de Fourier (IRTF) :

Les échantillons solides ont été analysés à l'aide d'un Spectromètre Nicolet de type Nexus. L'analyse est faite par réflexion totale atténuée (ATR) à l'aide d'un accessoire Smart Orbit de la société Thermo (accessoire ATR mono-réflection à cristal diamant). Le domaine spectral balayé est compris entre 4000 et 400 cm⁻¹ avec une résolution de 2 cm⁻¹ et un nombre de scan accumulé de 20.

### Exemple 1

On fait deux essais de dissolution d'environ 550 mg de (2R,3S)-3-ter-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle dans 14 g de Miglyol 812 Neutraloil Sasol. On met sous agitation magnétique à 500 tours par minute pendant 24 heures à température ambiante.

Après une semaine les échantillons sont filtrés sous vide et rincés à l'heptane. Chaque échantillon est analysé par DRXP pour confirmation de la forme obtenue. Après filtration on obtient entre 300 et 350 mg de forme anhydre D.

### Exemple 2

On dissout environ 3g de (2R,3S)-3-ter-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13a-yle dans un mélange 50 mL éthanol + 50 mL Polysorbate 80 pH 3.5. 100 mL d'eau sont ajoutés au précédent mélange et l'ensemble est homogénéisé. Après 48 heures de stockage à température ambiante des cristaux de forme anhydre D sont apparus. La quantité de produit cristallisé récupérée par filtration est d'environ 2,45g.

Une étude de stabilité comparative a été effectuée entre la forme A solvat à l'acétone et la forme anhydre D. La comparaison des analyses DRXP réalisées sur les formes A et D dès fabrication et après maintien un mois à 40°C conduit aux résultats suivants :
- Forme A : désolvatation partielle conduisant à l'obtention d'un mélange des formes A solvat acétone et B anhydre.
- Forme D : aucune évolution détectée après un mois de maintien à 40°C.

## Revendications

1. Forme anhydre D du (2R,3S)-3-ter-butoxycarbonylamino-2-hydroxy-3-phénylpropionate de 4a-acétoxy-2α-benzoyloxy-5β,20-époxy-1β-hydroxy-7β,10β-diméthoxy-9-oxo-tax-11-ène-13a-yle **caractérisée par** un diagramme DRXP présentant des raies caractéristiques situées à 3.9, 7.7, 7.8, 7.9, 8.6, 9.7, 10.6, 10.8, 11.1 et 12.3 ± 0.2 degrés 2-Thêta.

2. Procédé de préparation de la forme anhydre D selon la revendication 1 par cristallisation à température ambiante de la forme A solvat à l'acétone dans une huile suivie d'un rinçage avec de l'heptane.

3. Procédé de préparation de la forme anhydre D selon la revendication 1 par cristallisation pendant 48 heures à température ambiante d'une solution du (2R,3S)-3-tert-butoxycarbonylamino-2-hydroxy-3-phénylpropionate de 4α-acétoxy-2α-benzoyloxy-5β,20-époxy-1β-hydroxy-7β,10β-diméthoxy-9-oxo-tax-11-ène-13α-yle dans un mélange de polysorbate 80 pH 3.5, d'éthanol et d'eau.

4. Procédé selon la revendication 3, dans lequel le mélange de polysorbate 80 pH 3.5, d'éthanol et d'eau est un mélange 25/25/50.

## Patentansprüche

1. Wasserfreie Form D von (2R,3S)-3-tert-Butoxy-carbonylamino-2-hydroxy-3-phenylpropionsäure-4α-acetoxy-2α-benzoyloxy-5β,20-epoxy-1β-hydroxy-7β,10β-dimethoxy-9-oxotax-11-en-13α-ylester, **gekennzeichnet durch** ein XRPD-Diagramm mit charakteristischen Linien bei 3,9, 7,7, 7,8, 7,9, 8,6, 9,7, 10,6, 10,8, 11,1 und 12,3±0,2 Grad 2-Theta.

2. Verfahren zur Herstellung der wasserfreien Form D nach Anspruch 1 durch Kristallisation der Acetonsolvat-Form A aus einem Öl bei Umgebungstemperatur und anschließendes Waschen mit Heptan.

3. Verfahren zur Herstellung der wasserfreien Form D nach Anspruch 1 durch Kristallisation einer Lösung von (2R,3S)-3-tert-Butoxycarbonylamino-2-hydroxy-3-phenylpropionsäure-4α-acetoxy-2α-benzoyloxy-5β,20-epoxy-1β-hydroxy-7β,10β-dimethoxy-9-oxotax-11-en-13α-ylester in einer Mischung von Polysorbat 80 pH 3,5, Ethanol und Wasser über einen Zeitraum von 48 Stunden bei Umgebungstemperatur.

4. Verfahren nach Anspruch 3, wobei es sich bei der Mischung von Polysorbat 80 pH 3,5, Ethanol und Wasser um eine 25/25/50-Mischung handelt.

## Claims

1. Anhydrous form D of 4α-acetoxy-2α-benzoyloxy-5β,20-epoxy-1β-hydroxy-7β,10β-dimethoxy-9-oxotax-11-en-13α-yl (2R,3S)-3-tert-butoxycarbonylamino-2-hydroxy-3-phenylpropionate, **characterized by** a PXRD diagram exhibiting characteristic lines located at 3.9, 7.7, 7.8, 7.9, 8.6, 9.7, 10.6, 10.8, 11.1 and 12.3 ± 0.2 degrees 2-Theta.

2. Process for the preparation of the anhydrous form D according to Claim 1 by crystallization at ambient temperature of the acetone solvate form A from an oil, followed by rinsing with heptane.

3. Process for the preparation of the anhydrous form D according to Claim 1 by crystallization for 48 hours at ambient temperature of a solution of 4α-acetoxy-2α-benzoyloxy-5β,20-epoxy-1β-hydroxy-7β,10β-dimethoxy-9-oxotax-11-en-13α-yl (2R,3S)-3-tert-butoxycarbonylamino-2-hydroxy-3-phenylpropionate in a mixture of polysorbate 80 pH 3.5, ethanol and water.

4. Process according to Claim 3, in which the mixture of polysorbate 80 pH 3.5, ethanol and water is a 25/25/50 mixture.
